# EUROPEAN PATENT APPLICATION

(11) **EP 4 636 780 A1**
(43) Date of publication of application: **22.10.2025**
(21) Application number: 23901770.0
(22) Date of filing: 24.10.2023
(51) Int. Cl.: G16H 50/20

(54) **ASTHMATIC DISEASE CONDITION ESTIMATION SERVER, ASTHMATIC DISEASE CONDITION ESTIMATION PROGRAM, AND ASTHMATIC DISEASE CONDITION ESTIMATION METHOD**

(30) Priority: 16.12.2022 JP 2022200914
(71) Applicant: Yamaguchi University, Yamaguchi 753-8511 (JP)
(72) Inventor: HAMADA Kazuki, Ube-shi, Yamaguchi 755-8505 (JP); MATSUNAGA Kazuto, Ube-shi, Yamaguchi 755-8505 (JP); ASAI Yoshiyuki, Ube-shi, Yamaguchi 755-8505 (JP)
(74) Representative: Mathys & Squire
(86) International application number: PCT/JP2023/038330
(87) International publication number: WO 2024/127820

(57) **Abstract**

To easily estimate a disease condition of a patient with asthma. An asthmatic disease condition estimation server (1) according to the present invention includes: a storage (2) that stores a plurality of clusters, into which patients with asthma are classified, and a plurality of disease conditions of asthma associated with each cluster; a reception unit (3) that receives symptom information indicating a symptom of a subject; a determination unit (4) that identifies the cluster to which the subject belongs among the plurality of clusters, based on the symptom information, and identifies the disease condition stored in the storage in association with the identified cluster; and an output unit (5) that outputs the identified disease condition as an estimated disease condition of the subject.

## Description

### [Technical Field]

The present invention relates to an asthmatic disease condition estimation server, an asthmatic disease condition estimation program, and an asthmatic disease condition estimation method.

### [Background Art]

Asthma is one of chronic respiratory diseases having the highest prevalence rate and morbidity rate on a worldwide scale. Asthma has various disease conditions. Further, patient responsiveness to asthma treatment varies from patient to patient. Thus, a uniform approach to the treatment of patients with asthma is not appropriate; instead, the practice of individualized medical care is desirable. The practice of individualized medical care for each patient requires a detailed evaluation of a disease condition for each patient. The disease condition of patients with asthma is evaluated by a computed tomography (CT) test, a pulmonary function test, a fractional exhaled NO test, and the like. However, these tests require the installation of testing equipment, expertise in the medical interpretation of the test results, and the like. Thus, medical institutions capable of performing these tests are limited.

The invention in which a phenotype of a disease condition mechanism of asthma is classified and a therapeutic agent is then selected by measuring and analyzing gene expression data (related to type 2 inflammation) about airway epithelial cells from patients with asthma has been proposed (for example, see PTL 1). However, the invention requires extraction of airway epithelial cells by bronchoscopy, and a gene expression analysis (transcriptome analysis). The bronchoscopy is accompanied by a risk of complications caused by invasive testing. Further, the gene expression analysis requires a high cost. Thus, the practical application of this invention in routine clinical settings is not straightforward.

### [Citation List]

### [Patent Literature]

[PTL 1] JP2011-523350 A

### [Summary of Invention]

### [Technical Problem]

The present invention is directed to providing an asthmatic disease condition estimation server, an asthmatic disease condition estimation program, and an asthmatic disease condition estimation method that are capable of easily estimating the disease condition of a patient with asthma.

### [Solution to Problem]

An asthmatic disease condition estimation server according to an aspect of the present invention includes: a storage that stores a plurality of clusters, into which patients with asthma are classified, and a plurality of disease conditions of asthma associated with each cluster; a reception unit that receives symptom information indicating a symptom of a subject; a determination unit that identifies the cluster to which the subject belongs among the plurality of clusters, based on the symptom information, and identifies the disease condition stored in the storage in association with the identified cluster; and an output unit that outputs the identified disease condition as an estimated disease condition of the subject.

### [Advantageous Effects of Invention]

The present invention is capable of easily estimating the disease condition of a patient with asthma.

### [Brief Description of Drawings]

[Fig. 1] Fig. 1 is a functional block diagram illustrating an embodiment of an asthmatic disease condition estimation server according to the present invention.
[Fig. 2] Fig. 2 is a schematic diagram illustrating an example of information to be stored in a storage included in the above-described asthmatic disease condition estimation server.
[Fig. 3] Fig. 3 is a UMAP plot of scores of responses to an asthma control questionnaire for each training patient.
[Fig. 4] Fig. 4 is the UMAP plot of Fig. 3 labeled with scores of shortness of breath.
[Fig. 5] Fig. 5 is a schematic diagram illustrating a group with severe symptoms on the UMAP plot of Fig. 4.
[Fig. 6] Fig. 6 is the UMAP plot of Fig. 3 labeled with scores of wheezing.
[Fig. 7] Fig. 7 is a schematic diagram illustrating a group with severe symptoms on the UMAP plot of Fig. 6.
[Fig. 8] Fig. 8 is the UMAP plot of Fig. 3 labeled with scores of morning symptoms.
[Fig. 9] Fig. 9 is a schematic diagram illustrating a group with severe symptoms on the UMAP plot of Fig. 8.
[Fig. 10] Fig. 10 is the UMAP plot of Fig. 3 labeled with scores of nighttime symptoms.
[Fig. 11] Fig. 11 is a schematic diagram illustrating a group with severe symptoms on the UMAP plot of Fig. 10.
[Fig. 12] Fig. 12 is the UMAP plot of Fig. 3 labeled with scores of activity restriction.
[Fig. 13] Fig. 13 is a schematic diagram illustrating a group with severe symptoms on the UMAP plot of Fig. 12.
[Fig. 14] Fig. 14 is a dendrogram illustrating a group of the training patients classified by a hierarchical clustering analysis by using the scores of the responses to the asthma control questionnaire for each training patient.
[Fig. 15] Fig. 15 is a schematic diagram illustrating five clusters identified on the dendrogram illustrated in Fig. 14.
[Fig. 16] Fig. 16 is the UMAP plot of Fig. 3 labeled with the five clusters in Fig. 15.
[Fig. 17] Fig. 17 is a schematic diagram illustrating a relationship between the UMAP plot of Fig. 16 and the above-described five clusters.
[Fig. 18] Fig. 18 is a table illustrating the mean score for each symptom in the responses to the asthma control questionnaire by the training patients in each of the above-described five clusters.
[Fig. 19] Fig. 19 is a table illustrating the correlation coefficient between each of the above-described symptoms and disease conditions of asthma.
[Fig. 20] Fig. 20 is a graph illustrating the relationship between the above-described five clusters and the %FEV1 value.
[Fig. 21] Fig. 21 is a graph illustrating the relationship between the above-described five clusters and the fractional exhaled NO value.
[Fig. 22] Fig. 22 is a schematic diagram of an input screen for responses to the asthma control questionnaire to be displayed on a terminal connected to the above-described asthmatic disease condition estimation server.
[Fig. 23] Fig. 23 is a schematic diagram of an output screen for an estimated disease condition to be displayed on the terminal connected to the above-described asthmatic disease condition estimation server.

### [Description of Embodiments]

Embodiments of an asthmatic disease condition estimation server, an asthmatic disease condition estimation program, and an asthmatic disease condition estimation method according to the present invention will now be described with reference to the drawings.

### Configuration of Asthmatic Disease Condition Estimation Server

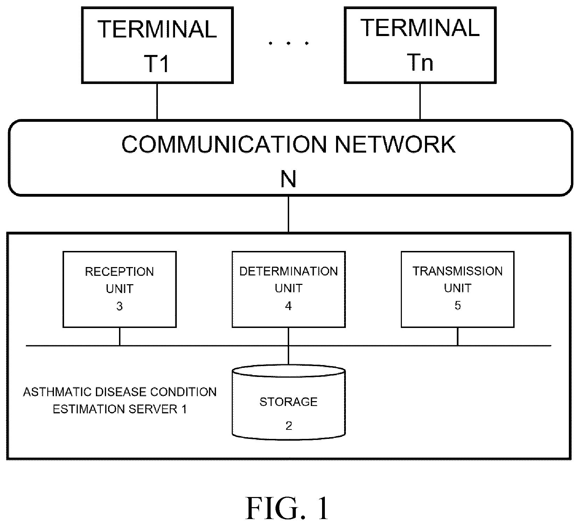

Fig. 1 is a functional block diagram illustrating an embodiment of the asthmatic disease condition estimation server according to the present invention (hereinafter referred to as "present server").

The present server is implemented by an information processing device such as a personal computer. The present server executes the asthmatic disease condition estimation program according to the present invention (hereinafter referred to as "present program"). The present program executed on the present server implements the asthmatic disease condition estimation method according to the present invention (hereinafter referred to as "present method") in cooperation with hardware resources of the present server.

The hardware resources of the present server are, for example, a processor such as a central processing unit (CPU), a micro processing unit (MPU), or a digital signal processor (DSP). The processor executes instructions described in the present program, thereby implementing means included in the present server described later.

Note that an information processing device (not illustrated) different from the present server executes the present program, and thus the information processing device functions similarly to the present server and implements the present method.

The present server 1 is communicably connected to a terminal T1, ..., and a terminal Tn via a communication network N.

The communication network in the present invention is a communication network such as the Internet or a local area network (LAN).

The terminal T1, ..., and the terminal Tn are information processing terminals that are installed in a medical facility and operated by a medical worker, or information processing terminals that are operated by a patient with asthma.

In the following description, each of the terminal T1, ..., and the terminal Tn is collectively referred to as a terminal T. In other words, the terminal T means all of the terminal T1, ..., and the terminal Tn.

The terminal T is implemented by a personal computer, a smartphone, or the like.

Herein, the present server 1 operates, for example, as a so-called web server. The terminal T operates, for example, as a so-called web client. The communication between the present server 1 and the terminal T is implemented by the present server 1 transmitting a web page to the terminal T in response to a request from the terminal T.

The present server 1 includes a storage 2, a reception unit 3, a determination unit 4, and a transmission unit 5.

The storage 2 stores the present program, information to be used by the present server 1 to implement the present method, and the like.

The types of the storage in the present invention are, for example, portable storage media such as a hard disk drive (HDD), a solid state drive (SSD), or a flash memory, other non-transitory recording media, or temporary recording media such as random access memory (RAM).

The reception unit 3 receives, as symptom information, responses of a subject to the asthma control questionnaire (ACQ) from the terminal T.

The subject is a patient with asthma whose disease condition is estimated by the present server 1.

The ACQ is a globally widespread and publicly known evaluation tool of asthmatic symptom control. The ACQ is a questionnaire that requires responses in seven levels (from 0 point to 6 points) to a condition of each of five symptoms (shortness of breath, wheezing, morning symptoms, nighttime symptoms, and activity restriction) in asthma. The mean score, calculated by dividing the total score (ranging from 0 point to 30 points) of the score corresponding to the responses to each of the five symptom items in the ACQ by the number of symptoms "five", is calculated as the ACQ score. The calculated ACQ score is used in clinical settings to evaluate whether asthma symptoms are well controlled.

Note that the symptom information in the present invention is not limited to responses to the ACQ, and may be, for example, information that can be used to score the control state of asthma symptoms in a patient (information that can be handled in machine learning described below) such as responses to an asthma control test (ACT) or responses to an asthma quality of life questionnaire (AQLQ).

The determination unit 4 identifies a cluster to which the subject belongs, based on the symptom information of the subject received by the reception unit 3, and then identifies a disease condition (an estimated disease condition and a predicted disease condition described below) of asthma of the subject. Details of the cluster and the disease condition of asthma will be described below.

Herein, five clusters in the present invention are determined through machine learning based on responses to the ACQ from training patients as described below. In other words, the determination unit 4 functions as a classifier for the machine-learned clusters. That is, the determination unit 4 classifies the subject into any of the five clusters, based on input responses to the ACQ by the subject. Then, the determiner 4 identifies a disease condition corresponding to the cluster of the classified subject as an estimated disease condition of the subject.

The transmission unit 5 transmits, as the estimated disease condition or a predicted disease condition of the subject, the disease condition identified by the determination unit 4 to the terminal T that has transmitted the symptom information of the subject to the present server 1. The transmission unit 5 is one example of the output unit in the present invention.

Note that an aspect of the output of the estimated disease condition and the predicted disease condition by the output unit in the present invention may include, for example, display on a display (not illustrated) included in the present server or printing by a printer (not illustrated) included in the present server other than transmission to the terminal T.

### Information Stored in Storage 2

Among the information stored in the storage 2, the information used by the present server 1 to implement the present method includes relationships between clusters and disease conditions of asthma, and responses of a subject to the ACQ.

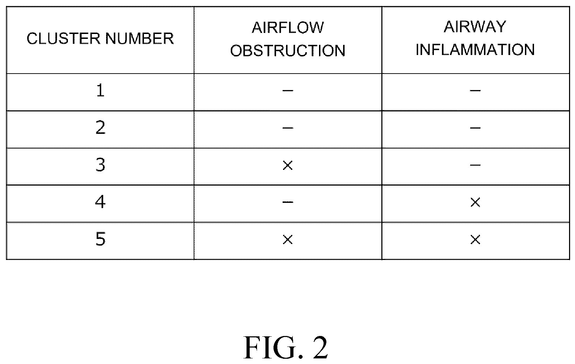

Fig. 2 is a schematic diagram illustrating the relationship between clusters and disease conditions stored in the storage 2. The figure illustrates that a corresponding disease condition is stored in association with each of the five clusters. **In** other words, for example, the figure illustrates that no disease condition corresponds to Clusters 1 and 2, a disease condition corresponding to Cluster 3 is airflow obstruction, a disease condition corresponding to Cluster 4 is airway inflammation, and a disease condition corresponding to Cluster 5 is airflow obstruction and airway inflammation.

Hereinafter, the clusters, the disease conditions, and the relationship between the clusters and the disease conditions in the present invention will be described.

The inventors of the present invention (hereinafter referred to as "present inventors") identified five clusters as asthmatic symptom phenotypes by performing unsupervised machine learning (dimensionality reduction and hierarchical clustering analysis described below) using responses to the ACQ from each of a plurality of (1,697) training patients. Then, the present inventors analyzed a correlation between the five clusters and two main disease conditions (airflow obstruction and airway inflammation) of asthma.

As described above, each of the answer choices corresponding to the ACQ responses is assigned a score ranging from 0 point to 6 points for each of the five asthma symptoms. That is, the ACQ score is expressed as a five-dimensional vector. Five-dimensional vector data is not visually interpretable. Thus, the present inventors reduced the ACQ score to two dimensions using a dimensionality reduction technique. The dimensionality reduction technique is uniform manifold approximation and projection (UMAP). Note that the dimensionality reduction technique in the present invention is not limited to the UMAP, and may be a publicly known technique such as a principal component analysis (PCA), t-distributed stochastic neighbor embedding (t-SNE), or pairwise controlled manifold approximation projection (PaCMAP).

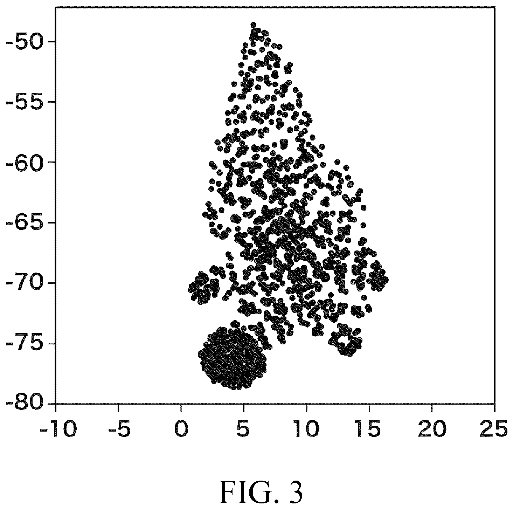

Fig. 3 is a UMAP plot of scores of responses to the ACQ for each training patient. The figure illustrates that the five-dimensional ACQ score vectors of the training patients are projected onto a two-dimensional plane. Each point in Fig. 3 represents a training patient (i.e., a case).

The present inventors visualized the ACQ scores by labeling the UMAP plot projected onto the two-dimensional plane with a score for each symptom of asthma.

A tool used for a UMAP analysis is umap-leam (version 0.5.3) being a library of Python3.

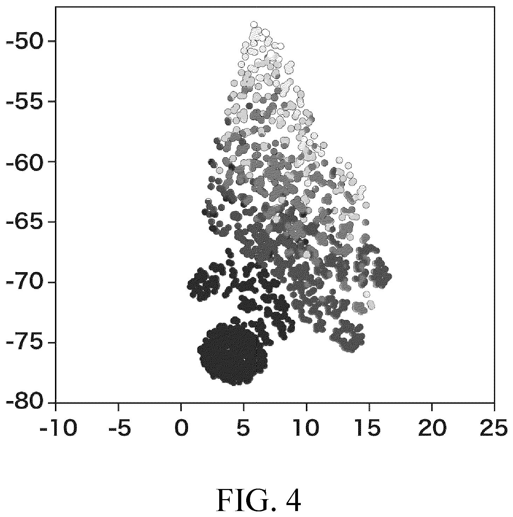

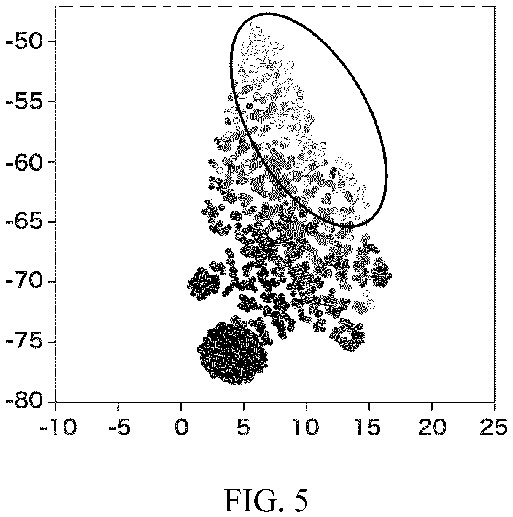

Fig. 4 is the UMAP plot of Fig. 3 labeled with scores of shortness of breath. The color differences among the dots in the figure represent differences in the scores (i.e., symptom severity), with lighter colors indicating higher scores (i.e., more severe symptoms). The same applies to Figs. 5 through 14.

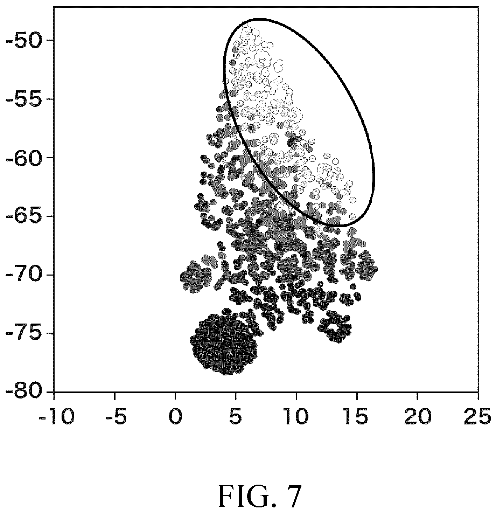

Fig. 5 is a schematic diagram illustrating a group with severe symptoms on the UMAP plot of Fig. 4. In the figure, a solid line indicates the group with severe symptoms. The same applies to Figs. 7, 9, 11, and 13.

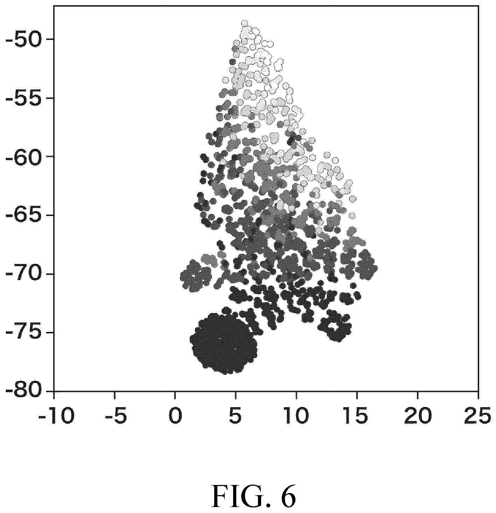

Fig. 6 is the UMAP plot of Fig. 3 labeled with scores of wheezing. Fig. 7 is a schematic diagram illustrating a group with severe symptoms on the UMAP plot of Fig. 6.

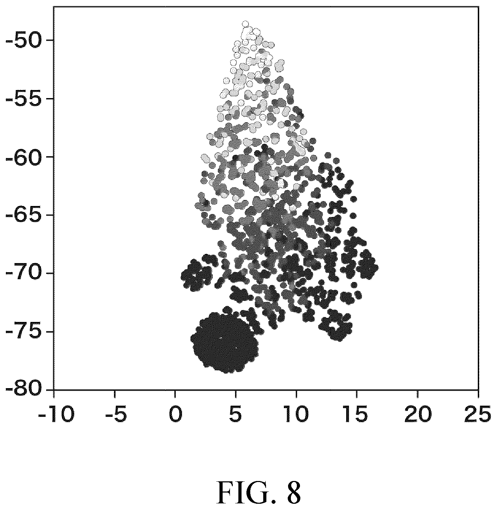

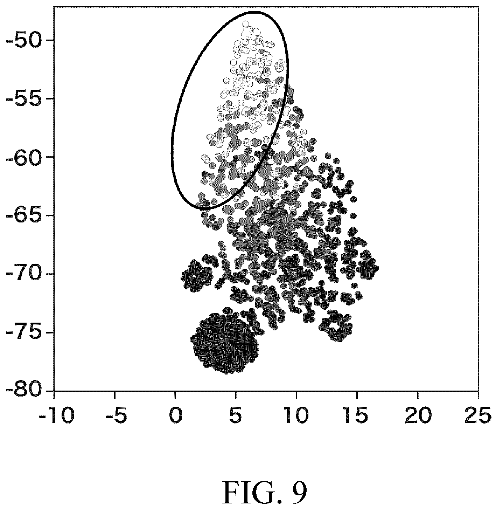

Fig. 8 is the UMAP plot of Fig. 3 labeled with scores of morning symptoms. Fig. 9 is a schematic diagram illustrating a group with severe symptoms on the UMAP plot of Fig. 8.

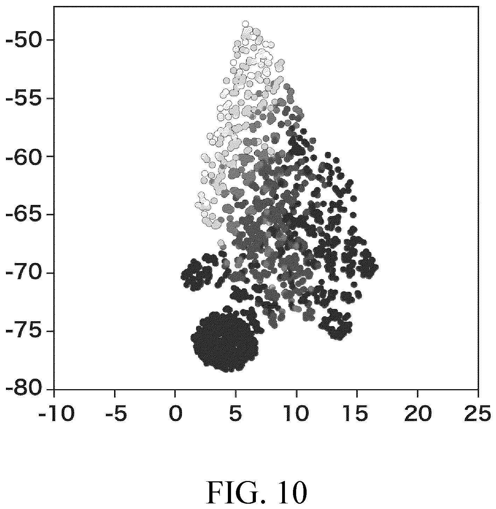

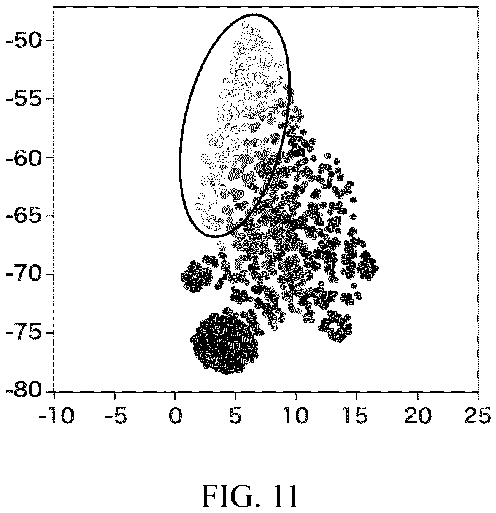

Fig. 10 is the UMAP plot of Fig. 3 labeled with scores of night symptoms. Fig. 11 is a schematic diagram illustrating a group with severe symptoms on the UMAP plot of Fig. 10.

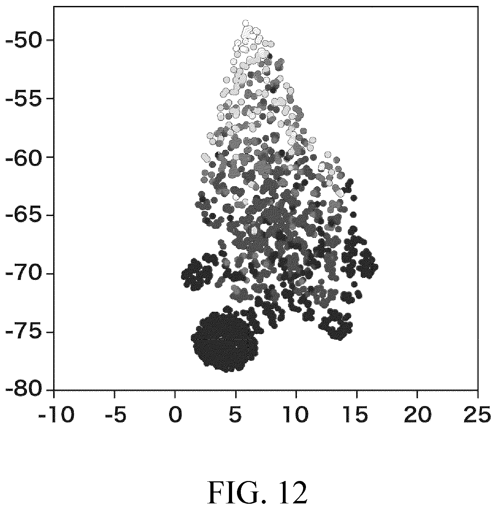

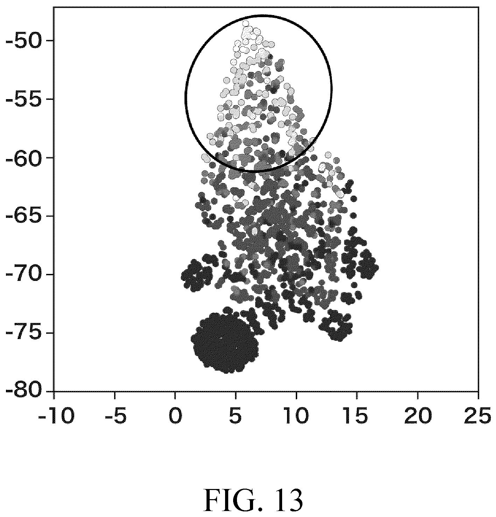

Fig. 12 is the UMAP plot of Fig. 3 labeled with scores of activity restriction. Fig. 13 is a schematic diagram illustrating a group with severe symptoms on the UMAP plot of Fig. 12.

Based on the UMAP plot labeled with the symptom-specific scores described above, the present inventors identified that the distribution of ACQ scores differs depending on the symptom.

Then, the present inventors performed the hierarchical clustering analysis by using the ACQ scores of the training patients.

A tool used for the hierarchical clustering analysis is scikit-learn (version 0.23.1). Euclidean distance was used to calculate the distance between vectors. Ward's method was used to measure the distance between clusters. The result of Ward's method is represented by a dendrogram based on dissimilarity.

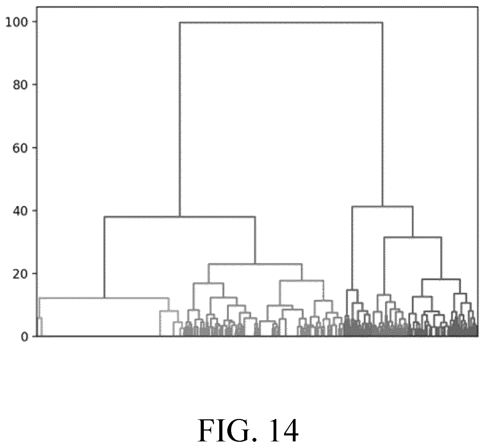

Fig. 14 is a dendrogram generated by performing the hierarchical clustering analysis using the ACQ scores of the training patients.

The present inventors determined the number of clusters to be "five", based on the dendrogram generated by performing the hierarchical clustering analysis illustrated in Fig. 14 and the distribution on the UMAP plot of the ACQ scores for each symptom illustrated in Figs. 3 to 13.

The determined number of clusters "five" is a minimum value that enables classification of patients with asthma (subjects) into any of the group indicated by a solid line in Fig. 5 or Fig. 7 (except for the group indicated by a solid line in Fig. 13), the group indicated by a solid line in Fig. 9 or Fig. 11 (except for the group indicated by the solid line in Fig. 13), the group indicated by the solid line in Fig. 13, or the other two groups. Herein, the solid line illustrated in each figure is for illustrative purposes to explain that the training patients represented on the UMAP plot in Fig. 3 belong to one of the five groups, and does not represent an exact boundary between the groups.

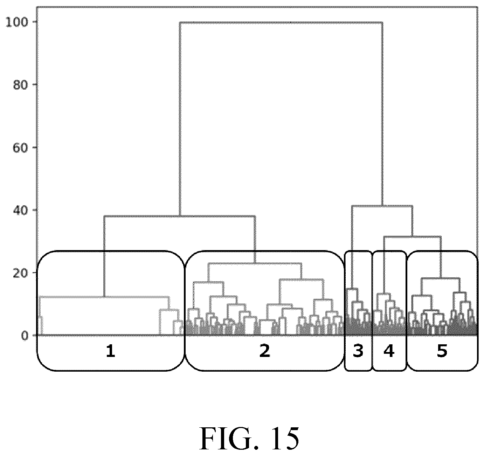

Fig. 15 is a schematic diagram illustrating the five clusters (Clusters 1, 2, 3, 4, and 5) on the dendrogram illustrated in Fig. 14.

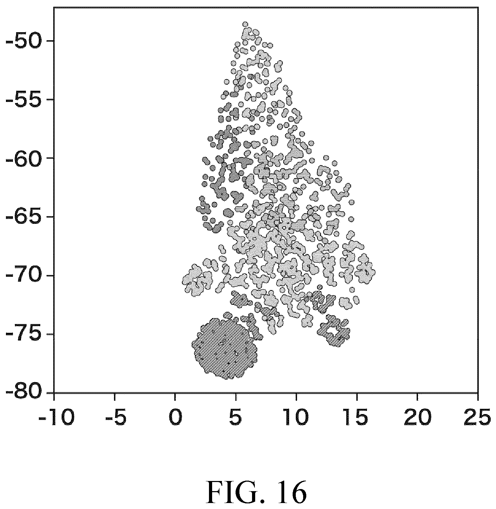

Fig. 16 is the UMAP plot of Fig. 3 labeled with the determined five clusters. In the figure, the color differences between the dots represent differences between clusters. In other words, dots with the same color belong to the same cluster.

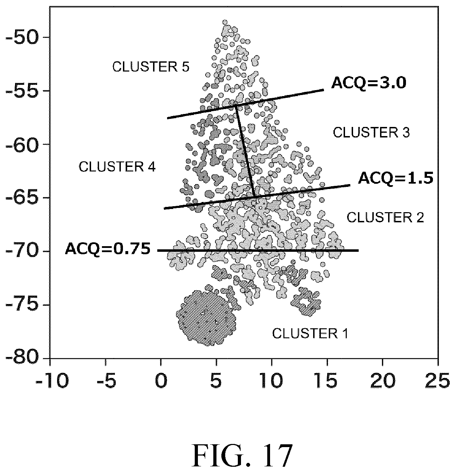

Fig. 17 is a schematic diagram illustrating a relationship between the UMAP plot of Fig. 16 and the determined five clusters. Four solid lines illustrated in the figure are for illustrative purposes to explain that the training patients represented on the UMAP plot in Fig. 16 belong to one of the five clusters, and do not represent an exact boundary between the clusters. The figure illustrates that a boundary between Cluster 1 and Cluster 2 is ACQ = 0.75. The figure illustrates that a boundary between Cluster 2 and Clusters 3 and 4 is ACQ = 1.5. The figure illustrates that a boundary between Clusters 3 and 4 and Cluster 5 is ACQ = 3.0.

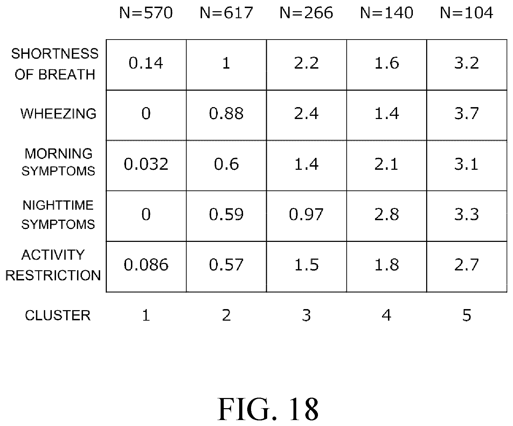

Fig. 18 is a table illustrating the mean ACQ score for each symptom of the training patients in each of the determined clusters. The figure illustrates that the number of training patients belonging to Clusters 1, 2, 3, 4, and 5 is 570, 617, 266, 140, and 104, respectively, for a total of 1,697 patients.

The figure illustrates that all of the ACQ scores for each of the five symptoms of the training patients belonging to Cluster 1 are lower than those of the training patients belonging to the other clusters. That is, the training patients belonging to Cluster 1 have almost no symptom in all of the five symptoms.

The figure illustrates that all of the ACQ scores for each of the five symptoms of the training patients belonging to Cluster 2 are low. That is, the training patients belonging to Cluster 2 have mild symptoms in all of the five symptoms.

The figure illustrates that the ACQ scores of "shortness of breath" and "wheezing" among the ACQ scores for each of the five symptoms of the training patients belonging to Cluster 3 are higher than the ACQ scores of the other symptoms. That is, "shortness of breath" and "wheezing" are severe among the symptoms of the training patients belonging to Cluster 3.

The figure illustrates that the ACQ scores of "moming symptoms" and "nighttime symptoms" among the ACQ scores for each of the five symptoms of the training patients belonging to Cluster 4 are higher than the ACQ scores of the other symptoms. That is, "morning symptoms" and "nighttime symptoms" are severe among the symptoms of the training patients belonging to Cluster 4.

The figure illustrates that all of the ACQ scores for each of the five symptoms of the training patients belonging to Cluster 5 are high. That is, the training patients belonging to Cluster 5 have severe symptoms in all of the five symptoms.

In this way, the present inventors identified the five clusters indicating a correlation with the symptoms of asthma using the ACQ scores of the training patients.

Then, the present inventors analyzed a correlation between the five clusters and disease conditions of asthma. Herein, the disease conditions of asthma being a target for the analysis are "airflow obstruction" and "airway inflammation" that are the main disease conditions.

The present inventors conducted an exhaustive correlation analysis between the five symptoms and airflow restriction (%FEV1 value) and the five symptoms and airway inflammation (fractional exhaled NO value), and calculated the correlation coefficients. The correlation coefficients are Spearman's rank correlation coefficients.

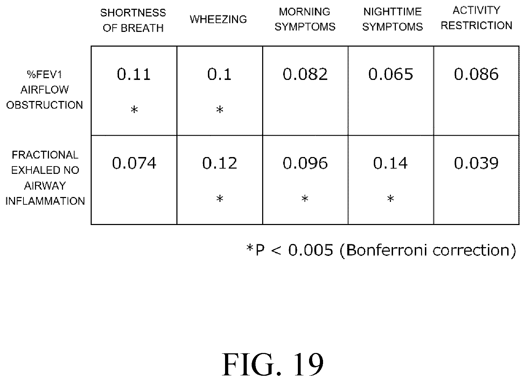

Fig. 19 is a table illustrating the correlation coefficients between the five symptoms and the two disease conditions, as calculated by the analysis conducted by the present inventors.

The figure illustrates that "shortness of breath" and "wheezing" have a negative correlation with the %FEV1 value. That is, the disease condition of asthma associated with "shortness of breath" and "wheezing" is airflow obstruction.

Fig. 19 illustrates that "moming symptoms" and "nighttime symptoms" have a positive correlation with the fractional exhaled NO values. That is, the disease condition of asthma associated with "moming symptoms" and "nighttime symptoms" is airway inflammation.

In order to clarify the treatable clinical traits (treatable traits) for each of the five clusters, the present inventors compared the degrees of airflow obstruction (%FEV1 value) and airway inflammation (fractional exhaled NO value) among the clusters.

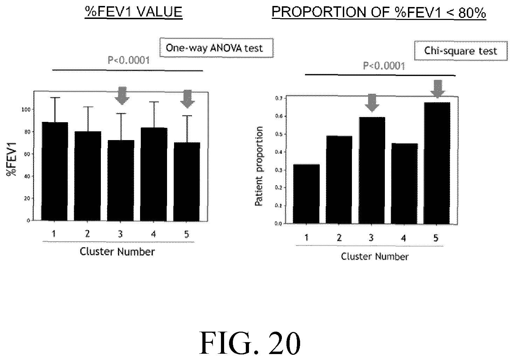

Fig. 20 is a graph illustrating the relationship between the five clusters and the %FEV1 value. The left figure illustrates the mean %FEV1 value for the training patients belonging to each cluster. The right figure illustrates the proportion of training patients belonging to each cluster whose %FEV1 value is less than 80% (used as a cutoff value in clinical settings) among the training patients belonging to each cluster. As illustrated in Fig. 20, it is indicated that the training patients belonging to Clusters 3 and 5 have a lower %FEV1 value being an index of airflow obstruction than that of the training patients belonging to the other clusters. In other words, the training patients belonging to Clusters 3 and 5 conceivably have a high risk of airflow obstruction.

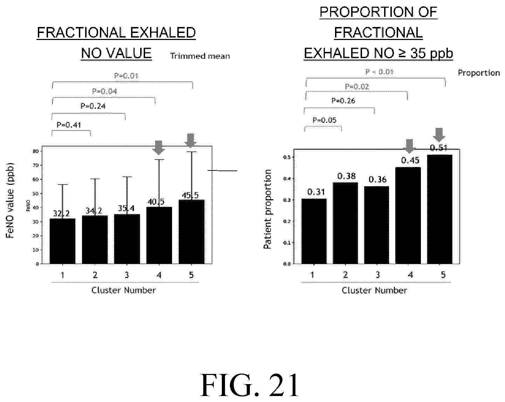

Fig. 21 is a graph illustrating a relationship between the five clusters and the fractional exhaled NO values. The left figure illustrates the mean fractional exhaled NO value for the training patients belonging to each cluster. The right figure illustrates the proportion of training patients belonging to each cluster whose fractional exhaled NO value is equal to or more than 35 ppb (used as a cutoff value in clinical settings) among the training patients belonging to each cluster. As illustrated in Fig. 21, it is indicated that the training patients belonging to Clusters 4 and 5 have a higher fractional exhaled NO value being an index of airway inflammation than that of the training patients belonging to the other clusters. In other words, the training patients belonging to Clusters 4 and 5 conceivably have a high risk of airway inflammation.

In this way, the present inventors identified a correlation between the five clusters and the disease conditions of asthma. As illustrated in Fig. 2, the relationship between the clusters and the disease conditions identified in this way is stored in the storage 2 and used to estimate disease conditions in subjects.

### Operation of Asthmatic Disease Condition Estimation Server

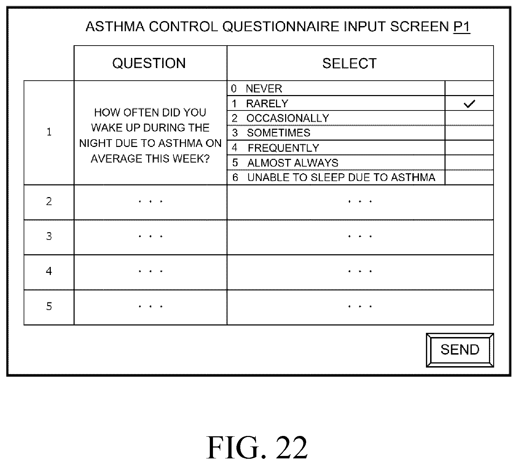

Fig. 22 is an input screen for responses to the ACQ to be displayed on the terminal T. The figure illustrates that a screen P1 displays seven selectable choices for each of five symptoms. In other words, for example, the figure illustrates that a second choice "1 RARELY" of the seven choices for a first symptom (shortness of breath) is selected. An operator of the terminal T finishes selecting a choice for each symptom displayed on the screen P1, and selects a button labeled "SEND" displayed on the screen P1, and thus information indicating the selected choice for each symptom selected on the screen P1 is transmitted from the terminal T to the present server 1.

Herein, the selection of choices and buttons on the screen P1 is performed through touch operations by the operator of the terminal T when, for example, the display of the terminal T is configured as a touch panel. Alternatively, the selection of choices and buttons on the screen P1 is performed by the operator of the terminal T using input means such as a mouse and a keyboard, which are included in the terminal T.

The present server 1 receives, as symptom information of a subject, the information transmitted from the terminal T using the reception unit 3, and stores the symptom information in the storage 2. Then, the present server 1 identifies, by using the determination unit 4, a cluster to which the subject belongs, based on the symptom information received from the terminal T, and identifies a disease condition corresponding to the identified cluster by referring to the information (see Fig. 2) stored in the storage 2. Then, the present server 1 transmits, as an estimated disease condition of the subject, the identified disease condition to the terminal T.

The terminal 1 receives the estimated disease condition of the subject transmitted from the present server 1, and allows the operator of the terminal T to view the estimated disease condition.

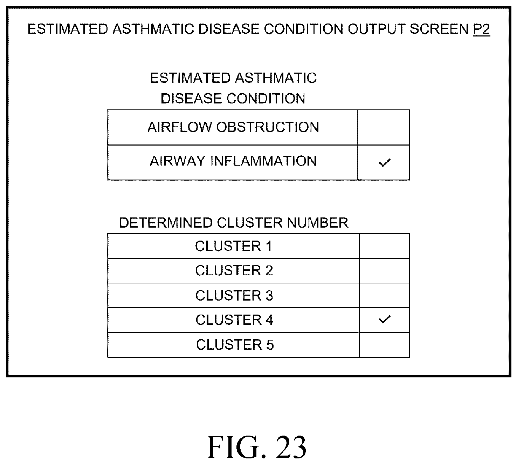

Fig. 23 is a schematic diagram of an output screen of the estimated disease condition displayed on the terminal T. The figure illustrates that an estimated disease condition "AIRWAY INFLAMMATION" and a determined cluster "4" are displayed on a screen P2. A doctor who views the screen P2 uses the estimated disease condition displayed on the screen P2 as reference information for determining a treatment plan, a therapeutic agent, a dosage of the therapeutic agent, and the like for the subject.

Herein, for example, when the present server 1 receives symptom information of the subject from the terminal T, the present server 1 may receive identification information (patient ID) for identifying the subject, together with the symptom information, and store the patient ID and the symptom information in association with each other in the storage 2. In other words, each time the present server 1 receives the symptom information of the same subject, the present server 1 stores the symptom information in association with the patient ID of the subject in the storage 2. The present server 1 uses the symptom information, stored in the storage 2 in association with the patient ID of the subject, as time-series symptom information of the subject in order to identify a predicted disease condition of the subject's information. In other words, the present server 1 is capable of identifying a latest (current) disease condition of the subject as an estimated disease condition only from latest symptom information, and is also capable of identifying a future disease condition of the subject as a predicted disease condition from the time-series symptom information.

The present server 1 may identify a predicted disease condition based on a change (transition) between the clusters to which the subject belongs, each cluster being identified for each piece of the time-series symptom information. In other words, for example, when the cluster to which the subject belongs changes from Cluster 1 to Cluster 2 over time, the subject may subsequently transition to Cluster 3 or 4. Thus, the present server 1 predicts a future cluster, based on the past transitions between clusters, and identifies a disease condition corresponding to the predicted cluster as the predicted disease condition.

The present server 1 may plot the time-series symptom information of the subject on the UMAP plot illustrated in Fig. 3 as individual cases of the subject, and may identify a predicted disease condition of the subject based on the trajectory of the cases of the subject on the UMAP plot. **In** other words, for example, when the latest case of the subject is located within Cluster 2 on the UMAP plot, a cluster to which the subject may belong in the future may be identified based on whether the trajectory formed by the time-series cases up to that point is directed toward Cluster 3 or Cluster 4, and a disease condition corresponding to the identified cluster may be identified as a predicted disease condition.

### Conclusion

According to the embodiments described above, a strong correlation is identified between the five clusters determined by machine learning using ACQ scores of training patients and two disease conditions of asthma. Thus, the present invention enables accurate estimation of a disease condition of a patient with asthma who is a subject to be evaluated, based on the ACQ score of the patient.

Herein, information needed to estimate a disease condition according to the present invention is responses by the subject to the ACQ that is currently widespread, that is, only a remaining symptom of the subject. **In** other words, unlike conventional techniques, the estimation of a disease condition according to the present invention requires neither large-scale specialized testing equipment nor experts, and it involves no risk of complications caused by invasive testing. As a result, the estimation of a disease condition according to the present invention is more easily implemented in routine clinical settings than that of conventional techniques.

Asthma is a chronic disease in which symptoms may persist in some patients (i.e., the disease does not go into remission) despite standard treatment. Thus, the effectiveness of asthma treatment requires continuous evaluation based on persistent symptoms, which are diverse and constantly changing. As described above, the present invention is capable of predicting a future disease condition of a subject, based on time-series responses to the ACQ by the subject. As a result, the present invention is capable of supporting a doctor in selection of a treatment plan, a therapeutic agent, a dosage of the therapeutic agent, and the like for the subject, based on the predicted disease condition, unlike conventional treatment based on residual symptoms at the present time.

### Features of Present Server, Present Program, and Present Method

Main characteristics of the present server, the present program, and the present method described above will be collectively described below.

### Features of Present Server

The present server includes: a storage (for example, the storage 2) configured to store a plurality of clusters (for example, Clusters 1, 2, 3, 4, and 5 in Fig. 2) into which patients with asthma are classified, and a plurality of disease conditions of asthma (for example, airflow obstruction and airway inflammation in Fig. 2) associated with each cluster; a reception unit (for example, the reception unit 3) configured to receive symptom information (for example, responses to the ACQ in Fig. 22) indicating a symptom of a subject; a determination unit (for example, the determination unit 4) configured to identify the cluster (for example, Cluster 4 in Fig. 23) to which the subject belongs among the plurality of clusters, based on the symptom information, and identify the disease condition stored in the storage in association with the identified cluster; and an output unit (for example, the transmission unit 5) configured to output the identified disease condition as an estimated disease condition (for example, airway inflammation in Fig. 23) of the subject.

**In** the present server, the plurality of disease conditions may include airway inflammation, and airflow obstruction, and the plurality of clusters may include an airway inflammation cluster (for example, Cluster 3) associated with only the airway inflammation of the airway inflammation and the airflow obstruction, an airflow obstruction cluster (for example, Cluster 4) associated with only the airflow obstruction of the airway inflammation and the airflow obstruction, and an all-disease condition cluster (for example, Cluster 5) associated with both of the airway inflammation and the airflow obstruction.

In the present server, the reception unit may receive, as the symptom information, responses to an asthma control questionnaire (for example, the ACQ) by the subject.

In the present server, the plurality of clusters may correspond to each group (for example, the group in Figs. 5 and 7 and the group in Figs. 9 and 11) of a plurality of training patients having a similar pattern of the symptom, based on responses to the asthma control questionnaire by each of the training patients.

In the present server, the group of the training patients may be determined by performing machine learning (for example, dimensionality reduction and hierarchical clustering analysis) on the responses from each of the plurality of training patients.

In the present server, the determination unit may identify the cluster for each piece of the time-series symptom information of the subject, identify, as a predicted cluster, the cluster to which the subject is predicted to belong, based on the identified cluster for each piece of the time-series symptom information, and identify, as a predicted disease condition, the disease condition stored in the storage in association with the predicted cluster.

### Features of Present Program

The present program causes a computer to function as the present server.

### Features of Present Method

The present method is an asthmatic disease condition estimation method executed by an information processing device (for example, the asthmatic disease condition estimation server 1) including a storage (for example, the storage 2) configured to store a plurality of clusters (for example, Clusters 1, 2, 3, 4, and 5 in Fig. 2) into which patients with asthma are classified, and a plurality of disease conditions of asthma (for example, airflow obstruction and airway inflammation in Fig. 2) associated with each cluster, and the asthmatic disease condition estimation method includes: a reception step of receiving, by the information processing device, symptom information (for example, responses to the ACQ in Fig. 22) indicating a symptom of a subject; a determination step of identifying, by the information processing device, the cluster to which the subject belongs among the plurality of clusters, based on the symptom information, and identifying the disease condition stored in the storage in association with the identified cluster; and an output step of outputting, by the information processing device, the identified disease condition as an estimated disease condition (for example, airway inflammation in Fig. 23) of the subject.

### [Reference signs List]

- 1: Asthmatic disease condition estimation server
- 2: Storage
- 3: Reception unit
- 4: Determination unit
- 5: Transmission unit
- T: Terminal
- P1: Input screen (asthma control questionnaire)
- P2: Output screen (estimated asthmatic disease condition)

## Claims

1. An asthmatic disease condition estimation server comprising:
a storage configured to store a plurality of clusters into which patients with asthma are classified, and a plurality of disease conditions of asthma associated with each cluster;
a reception unit configured to receive symptom information indicating a symptom of a subject;
a determination unit configured to identify the cluster to which the subject belongs among the plurality of clusters, based on the symptom information, and identify the disease condition stored in the storage in association with the identified cluster; and
an output unit configured to output the identified disease condition as an estimated disease condition of the subject.

2. The asthmatic disease condition estimation server according to claim 1, wherein
the plurality of disease conditions includes
airway inflammation, and
airflow obstruction, and
the plurality of clusters includes
an airway inflammation cluster associated with only the airway inflammation of the airway inflammation and the airflow obstruction,
an airflow obstruction cluster associated with only the airflow obstruction of the airway inflammation and the airflow obstruction, and
an all-disease condition cluster associated with both of the airway inflammation and the airflow obstruction.

3. The asthmatic disease condition estimation server according to claim 2, wherein the reception unit receives, as the symptom information, responses to an asthma control questionnaire by the subject.

4. The asthmatic disease condition estimation server according to claim 3, wherein the plurality of clusters corresponds to each group of a plurality of training patients having a similar pattern of the symptom, based on responses to the asthma control questionnaire by each of the training patients.

5. The asthmatic disease condition estimation server according to claim 4, wherein the group of the training patients is determined by performing machine learning on the responses from each of the plurality of training patients.

6. The asthmatic disease condition estimation server according to claim 5, wherein
the determination unit is configured to
identify the cluster for each piece of the time-series symptom information of the subject,
identify, as a predicted cluster, the cluster to which the subject is predicted to belong, based on the identified cluster for each piece of the time-series symptom information, and
identify, as a predicted disease condition, the disease condition stored in the storage in association with the predicted cluster.

7. An asthmatic disease condition estimation program causing a computer to function as the asthmatic disease condition estimation server according to claim 1.

8. An asthmatic disease condition estimation method executed by an information processing device including a storage configured to store a plurality of clusters into which patients with asthma are classified, and a plurality of disease conditions of asthma associated with each cluster, the asthmatic disease condition estimation method comprising:
a reception step of receiving, by the information processing device, symptom information indicating a symptom of a subject;
a determination step of identifying, by the information processing device, the cluster to which the subject belongs among the plurality of clusters, based on the symptom information, and identifying the disease condition stored in the storage in association with the identified cluster; and
an output step of outputting, by the information processing device, the identified disease condition as an estimated disease condition of the subject.
